(19) **Europäisches Patentamt European Patent Office Office européen des brevets**

(11) **EP 4 333 782 B1**

(12) **FASCICULE DE BREVET EUROPEEN**

(45) Date de publication et mention de la délivrance du brevet:
**03.06.2026 Bulletin 2026/23**

(21) Numéro de dépôt: **22726146.8**

(22) Date de dépôt: **06.05.2022**

(51) Classification Internationale des Brevets (IPC):
**A61F 9/009** (2006.01)  **A61F 9/008** (2006.01)

(52) Classification Coopérative des Brevets (CPC):
**A61F 9/009;** A61F 9/00825; A61F 2009/00872

(86) Numéro de dépôt international:
**PCT/EP2022/062236**

(87) Numéro de publication internationale:
**WO 2022/234060 (10.11.2022 Gazette 2022/45)**

(54) **INTERFACE DE COUPLAGE ENTRE UNE SOURCE L.A.S.E.R. ET UN TISSU À TRAITER**

KOPPELSCHNITTSTELLE ZWISCHEN EINER LASERQUELLE UND EINEM ZU BEHANDELNDEN GEWEBE

COUPLING INTERFACE BETWEEN A LASER SOURCE AND A TISSUE TO BE TREATED

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.05.2021 FR 2104803**

(43) Date de publication de la demande:
**13.03.2024 Bulletin 2024/11**

(73) Titulaire: **KERANOVA**
**42000 Saint Etienne (FR)**

(72) Inventeurs:
• **ROMANO, Fabrizio**
  **01700 BEYNOST (FR)**
• **BERNARD, Aurélien**
  **42100 SAINT ETIENNE (FR)**

(74) Mandataire: **Be IP**
**Cabinet LTL SAS**
**Centre d'Entreprise et d'Innovation**
**56, Bd Niels Bohr**
**CS 52132**
**69603 Villeurbanne Cedex (FR)**

(56) Documents cités:
WO-A1-2018/154038    WO-A1-2019/104256
CN-B- 111 920 579    DE-A1- 102005 040 338
US-A1- 2014 216 468

EP 4 333 782 B1

## Description

## DOMAINE TECHNIQUE

La présente invention concerne le domaine technique des opérations chirurgicales réalisées au L.A.S.E.R., et plus particulièrement celui de la chirurgie ophtalmologique pour notamment des applications de découpe de cornée, ou de cristallin.

L'invention concerne une interface d'adaptation permettant le couplage d'une source L.A.S.E.R. au tissu humain ou animal devant être traité, tel qu'une cornée, ou un cristallin.

Par « *source L.A.S.E.R.* », on entend une source lumineuse, apte à émettre un faisceau L.A.S.E.R. sous forme d'impulsions ultra-courtes, dont la durée est comprise entre 1 femtoseconde et 100 picosecondes, de préférence comprise entre 1 et 1000 femtosecondes, notamment de l'ordre de la centaine de femtosecondes.

## ART ANTERIEUR

Il est connu de l'état de la technique de réaliser des opérations chirurgicales de l'œil au moyen d'une source L.A.S.E.R., telles que des opérations de découpe de cornée ou de cristallin.

Une source L.A.S.E.R. est un instrument apte à réaliser une découpe du tissu cornéen par exemple, en focalisant un faisceau L.A.S.E.R. dans le stroma de la cornée, et en réalisant une succession de petites bulles de cavitation adjacentes, qui forment ensuite une ligne de découpe.

Plus précisément, lors de la focalisation du faisceau L.A.S.E.R. dans la cornée, un plasma est généré par ionisation non-linéaire lorsque l'intensité de la source L.A.S.E.R. dépasse une valeur seuil, nommée seuil de claquage optique. Une bulle de cavitation se forme alors, engendrant une disruption très localisée des tissus environnants. Ainsi, le volume réellement ablaté par la source L.A.S.E.R. est très faible comparativement à la zone disruptée.

Pour permettre le couplage de la source L.A.S.E.R. à l'œil d'un patient, il est connu d'utiliser une interface d'adaptation disposée entre l'œil du patient et la source L.A.S.E.R..

Une telle interface de couplage permet de maintenir le globe oculaire dans une position stable et constante, centrée et à une distance connue de la source L.A.S.E.R., en évitant tout mouvement, pendant la durée d'un traitement.

### 1. *Interface de couplage connue de l'art antérieur*

### 1.1. *Premier exemple d'interface de couplage existante*

### 1.1.1. *Description générale*

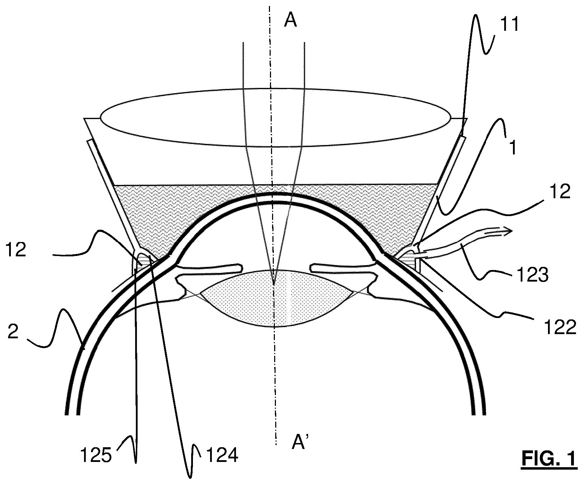

En référence à la figure 1, on a illustré un premier exemple d'interface de couplage de l'art antérieur. L'interface de couplage comprend un anneau incluant un tronc de cône 1 d'axe A-A'. Le tronc de cône 1 est ouvert à ses deux extrémités. La grande base 11 du tronc de cône 1 est destinée à recevoir l'extrémité de la source L.A.S.E.R.. La petite base du tronc de cône 1 est destinée à venir en contact avec l'œil 2. L'interface de couplage comprend également :

- une gorge annulaire 12 de circulation de gaz s'étendant à la périphérie de la petite base, et
- un membre d'accès tubulaire 122 s'étendant radialement vers l'extérieur et permettant le raccordement de la gorge annulaire 12 à un dispositif d'aspiration externe (non représenté) par l'intermédiaire d'une tubulure de connexion 123.

La gorge annulaire 12 présente une forme de U en section définie par deux bordures circulaires 124, 125 destinées à être plaquées contre l'œil 2 du patient. Le dispositif d'aspiration est connu en soi par l'homme du métier et permet de créer une dépression dans la gorge annulaire 12 une fois les bordures circulaires 124, 125 appliquées sur l'œil 2. La génération d'une dépression dans la gorge annulaire 12 permet de solidariser l'interface de couplage sur l'œil 2 par effet ventouse pendant toute la durée du traitement.

### 1.1.2. *Principe de fonctionnement du premier exemple d'interface de couplage existante*

Le principe de fonctionnement d'une telle interface de couplage est le suivant.

Dans un premier temps, le praticien dispose l'anneau de l'interface de couplage sur l'œil 2.

Une fois l'anneau correctement positionné et centré, le dispositif d'aspiration est activé pour générer une dépression dans l'espace défini entre la gorge annulaire 12 et l'œil 2 afin de solidariser l'interface de couplage sur l'œil 2 par ventousage.

Lorsque l'anneau est solidaire de l'œil 2, le cône 1 est rempli d'un liquide ayant un indice de réfraction proche de celui de la cornée jusqu'à immersion de la cornée. Le liquide immergeant ainsi la cornée, a l'avantage de simplifier la trajectoire des faisceaux L.A.S.E.R. à travers la cornée en effaçant optiquement le dioptre cornéen.

La source L.A.S.E.R. est ensuite placée au droit de l'interface de couplage, la partie distale de la source L.A.S.E.R. étant insérée et verrouillée sur l'interface de couplage, de manière à ce que l'œil 2 et l'axe optique du L.A.S.E.R. soient alignés et maintenus solidairement pendant la durée du traitement.

### 1.1.3. *Inconvénients du premier exemple d'interface de couplage existante*

Une telle interface de couplage présente de nombreux inconvénients.

### 1.1.3.1.*Pression exercée sur l'interface pour assurer l'étanchéité*

L'efficacité du ventousage de l'interface de couplage sur l'œil 2 dépend de la qualité de l'étanchéité entre les bordures circulaires 124, 125 et l'œil 2.

Un contact parfait, doit donc être obtenu sur toute la circonférence de la ligne de contact de chaque bordure circulaire 124, 125.

Ceci n'est pas toujours simple, les yeux des patients étant tous différents, la surface du globe ne suit pas forcément la pente entre chaque bordure circulaire 124, 125.

Pour pallier cette inhomogénéité entre les yeux des patients, il est donc nécessaire d'appuyer fortement l'anneau de l'interface de couplage pour obtenir une bonne étanchéité entre la gorge 12 et l'œil 2, ce qui est préjudiciable au confort et à la sécurité du patient. En effet, la force exercée sur l'anneau de l'interface de couplage pour obtenir une étanchéité satisfaisante tend à faire pénétrer les bordures circulaires 124, 125 dans la conjonctive.

La force à appliquer pour obtenir l'étanchéité est parfois si importante que l'inconfort du patient peut tendre vers la douleur. Par ailleurs, cette pression, même si de courte durée, provoque immédiatement l'augmentation de la pression intraoculaire du patient, ce qui chez certains patients à risque (forts myopes, antécédents de décollement de rétine ou glaucome), peut se traduire par des effets secondaires graves.

### 1.1.3.2.*Intensité de la dépression*

L'intensité de la dépression doit être importante pour assurer le maintien en position de l'interface de couplage.

En effet, pour résister aux micromouvements oculaires et à la tendance au dé-ventousage, la force d'aspiration doit être importante.

Or il est connu que la Force F d'aspiration est fonction de la Dépression P et de la Surface S sur laquelle est appliquée cette dépression, soit :

$$F = P \times S.$$

Dans les interfaces de couplage traditionnelles, la surface sur laquelle est appliquée la dépression étant faible (i.e. limitée à la surface de l'œil entre les deux bordures circulaires), il est nécessaire d'appliquer une dépression de valeur importante pour obtenir une force d'aspiration F permettant de résister aux micromouvements oculaires et à la tendance au dé-ventousage.

L'importance de la dépression appliquée entraîne parfois l'apparition d'hémorragies conjonctivales (effusion de globules rouges liée à l'aspiration sous la conjonctive). Même si des ecchymoses superficielles sont généralement très limitées, et n'ont aucune conséquence sur la vision, ce sont toujours une source d'inquiétude pour les patients et sont assez inesthétiques et il est préférable de limiter leur apparition.

### 1.1.3.3.*Coût*

Certaines interfaces de couplage traditionnelles fonctionnent avec des équipements chirurgicaux spécifiques. Ceux-ci ont dû intégrer un système de ventousage, avec pompe, tubulures et capteur de dépression. Chaque système étant spécifique, il est nécessaire d'acheter tous ces accessoires. La présente invention évite de faire cette dépense inutile. En effet, cette interface patient fonctionne avec des accessoires déjà présents au bloc opératoire, et notamment l'appareil de phacoémulsification. Celui-ci est équipé d'une pompe d'aspiration avec capteur de dépression et d'une ligne d'infusion, et dispose déjà des tubulures adéquates. Nos essais expérimentaux démontrent que la nouvelle interface de couplage, objet de cette invention, fonctionne parfaitement avec un appareil de phacoémulsification, ce qui génère une économie substantielle pour le client.

### 1.2.*Deuxième exemple d'interface de couplage existante*

Pour remédier aux inconvénients précités, le document WO2018154038 propose une interface de couplage comprenant :

- un anneau incluant une paroi latérale, une extrémité proximale destinée à venir en contact avec le tissu à traiter, et une extrémité distale destinée à recevoir l'extrémité de la source L.A.S.E.R.,
- une fenêtre transparente à un faisceau L.A.S.E.R. généré par la source L.A.S.E.R., ladite fenêtre étant montée de manière étanche sur l'anneau de sorte à fermer l'extrémité distale de l'anneau,
- un canal d'aspiration traversant s'étendant entre les faces interne et externe de la paroi latérale, le canal d'aspiration étant destiné à être connecté à un module d'aspiration pour permettre la génération d'une dépression dans un espace intérieur défini entre la fenêtre , la paroi latérale, et la partie supérieure du globe oculaire,
- un canal d'irrigation traversant s'étendant entre les faces interne et externe de la paroi latérale, le canal d'irrigation étant destiné à être connecté à un module d'irrigation pour permettre l'infusion de liquide dans l'espace intérieur.

En générant une dépression dans tout l'espace intérieur de l'interface de couplage, la surface sur laquelle la dépression agit est considérablement augmentée par rapport à l'interface de couplage illustrée à la figure 1 (dans laquelle le vide est appliqué uniquement au niveau d'une bordure circulaire). Le niveau de vide s'appliquant sur l'œil est alors réduit, ce qui contribue à une réduction des effets secondaires dus à une dépression trop élevée. La procédure de couplage devient plus agréable pour le patient.

Même si cette interface présente de nombreux avantages, l'œil du patient peut bouger d'un ou plusieurs dizaines de microns. Or, pour la réalisation de certaines découpes, il est préférable que la cornée reste totalement immobile.

Le document DE 10 2005 040338 décrit un adaptateur permettant de coupler mécaniquement un dispositif de traitement laser à l'œil d'un patient. L'adaptateur selon DE 10 2005 040338 comprend : un corps de lentille en verre ayant une face inférieure adaptée à la courbure de la cornée humaine, et un support entourant le corps de lentille.

Le document WO 2019/104256 décrit une interface patient pour un système d'irradiation de lentille intraoculaire réglable.

Un but de la présente invention est de proposer une nouvelle interface de couplage présentant les mêmes avantages que l'interface de couplage décrite dans WO2018154038, et permettant d'immobiliser totalement l'œil du patient (mouvement de l'œil inférieur à la dizaine de microns).

**EXPOSE DE L'INVENTION**

A cet effet l'invention propose une interface de couplage entre une source L.A.S.E.R. et un tissu à traiter, ***remarquable en ce que*** l'interface de couplage comprend :

- un anneau ayant une extrémité proximale destinée à venir en contact avec le tissu à traiter et une extrémité distale opposée, l'anneau incluant :

  • une cloison à double-paroi comportant :

    ▪ une paroi latérale (cylindrique) interne ayant un bord supérieur et un bord inférieur et
    ▪ une paroi latérale (cylindrique) externe ayant un bord supérieur et un bord inférieur,

    le bord inférieur de la paroi latérale interne étant plus proche de l'extrémité distale que le bord inférieur de la paroi latérale externe, et les bords supérieurs des parois latérales interne et externe étant plus proches de l'extrémité distale que les bords inférieurs des parois latérales interne et externe,

  • un disque perforé s'étendant entre les bords inférieurs des parois latérales interne et externe, le disque incluant des lumières traversantes,

  • une fenêtre transparente à un faisceau L.A.S.E.R. généré par la source L.A.S.E.R., ladite fenêtre étant montée de manière étanche sur les bords supérieurs des parois latérales interne et externe de sorte à fermer l'extrémité distale de l'anneau, la fenêtre définissant :

    ▪ une chambre d'aspiration périphérique avec le disque perforé, la paroi latérale interne et la paroi latérale externe, et
    ▪ un logement de travail central avec la paroi latérale interne, et

  • un canal d'aspiration s'étendant entre les faces intérieure et extérieure de la paroi latérale externe de sorte à déboucher dans la chambre d'aspiration (entre les parois interne et externe), le canal d'aspiration étant destiné à être connecté à un module d'aspiration pour permettre la génération d'une dépression dans un espace intérieur défini par la chambre d'aspiration,

- un bloc de gel destiné à être positionné dans le logement de travail, ledit bloc de gel ayant :

  • un flanc latéral de forme complémentaire à la forme de la paroi latérale interne, le flanc latéral étant destiné à venir en contact avec la face intérieure de la paroi latérale interne,
  • une base supérieure circulaire sensiblement plane, la base supérieure étant destinée à venir en contact avec la fenêtre,
  • une base inférieure circulaire et concave, la base inférieure étant destinée à venir en contact avec le tissu à traiter.

Le bloc de gel est monté de manière amovible dans le logement de travail. Par ailleurs, le bloc de gel est apte à coulisser le long de la paroi latérale interne de la double-cloison. Enfin, le bloc de gel est déformable.

L'interface de couplage décrite ci-dessus se distingue des interfaces existantes, notamment en ce qu'elle comprend un anneau incluant une cloison à double-paroi, une fenêtre montée sur les bords supérieurs de la cloison à double-paroi, un logement de travail délimité par la fenêtre et la paroi latérale interne de la cloison à double-paroi, et un bloc de gel monté de manière amovible dans le logement de travail.

La combinaison de ces caractéristiques permet de disposer d'une interface de couplage qui se conforme à la forme de l'oeil du patient, contrairement par exemple à l'adaptateur selon DE 10 2005 040338 (qui propose l'utilisation d'un corps de lentille en verre et) dans lequel c'est l'œil du patient qui est déformé (via l'application d'une force d'appui importante) pour se conformer à la forme de la face inférieure du corps de lentille en verre.

L'utilisation d'un bloc de gel permet de réduire les risques de déplacement de l'œil du patient durant la procédure chirurgicale. En effet, le coefficient de frottement de la surface de contact entre l'œil et le bloc de gel est supérieur au coefficient de frottement de la surface de contact entre l'œil et le liquide à indice de réfraction proche de celui de la cornée, tel que proposé dans le document WO2018154038. L'utilisation d'un bloc de gel permet également de réduire les risques de déplacement verticaux de la cornée (l'hydrogel limite mieux les mouvements verticaux que les fluides). Ceci est très important pour les incisions cornéennes non-transfixiante (telles que les incisions arciformes pour la correction d'un astigmatisme).

Pour autant, la présente invention ne se limite pas au remplacement d'un liquide de couplage par un bloc de gel. En effet, pour bénéficier des avantages associés au dispositif décrit dans WO2018154038, les inventeurs ont dû repenser toute la structure de l'interface de couplage selon WO2018154038.

Notamment, la présence d'une cloison à double-paroi permet d'éviter l'aspiration du bloc de gel au travers du canal d'aspiration. Elle permet en outre d'assurer le centrage du bloc de gel lors de la mise en place de l'interface patient.

Par ailleurs, la combinaison de la cloison à double-paroi et du bloc de gel permet de réduire l'inconfort pour le patient par rapport aux solutions de l'art antérieur telles qu'illustrées à la figure 1. En effet, dans l'interface illustrée à la figure 1, deux bordures circulaires 124, 125 (i.e. une bordure interne 124 et une bordure externe 125) sont destinées à venir en contact avec l'oeil du patient, ce qui oblige le praticien à appuyer fortement sur l'anneau pour garantir un contact parfait entre l'oeil du patient et chacune de ces bordures circulaires 124, 125. Dans le cadre de la présente invention, c'est le bloc de gel remplace la bordure interne. Ainsi, la cloison à double-paroi comporte une unique bordure destinée à venir en contact avec l'oeil du patient et l'obtention :

- d'un contact parfait entre le bloc de gel et l'oeil du patient est obtenu grâce à la déformabilité du bloc de gel, et
- d'un contact parfait entre l'unique bordure de la cloison à double-paroi et l'œil du patient ne nécessite pas l'application d'une force d'appui importante de la part de l'utilisateur (il est plus facile d'obtenir un contact parfait entre l'œil et une bordure circulaire unique-principe de l'anneau sur la sphère où on obtient toujours un contact à 360° - plutôt qu'entre l'œil et deux bordures concentriques comme proposé avec l'interface patient de l'art antérieur illustré à la figure 1).

Plus précisément, pour mettre en place l'interface patient illustrée à la figure 1, c'est l'œil du patient qui est déformé (via l'application d'une force d'appui importante) pour se conformer à la forme de l'interface patient ; au contraire avec la solution de la présente invention, c'est l'interface patient qui se conforme à la forme de l'œil du patient grâce à l'utilisation :

- d'une cloison à double-paroi ayant une unique bordure de contact avec l'œil du patient, et à l'utilisation

- d'un bloc de gel mobile déformable .

Une telle interface de couplage permet un ventousage rapide et indolore tout en garantissant l'immobilité de l'œil du patient. Cette interface de couplage est compatible avec l'utilisation d'un module d'aspiration, qui peut être intégré dans un appareil couramment présent en bloc opératoire ophtalmique : le phacoémulsificateur ou tout autre module d'aspiration aux performances adéquates équipé des capteurs nécessaires. Bien entendu, la présente invention est également compatible avec l'utilisation d'un module d'aspiration non intégré.

Plusieurs modèles peuvent être prévus pour le bloc de gel, la base inférieure circulaire concave de chaque modèle de bloc de gel ayant un rayon de courbure différent des rayons de courbure des bases inférieures circulaires concaves des autres modèles de blocs de gel.

En effet :

- la déformabilité du bloc de gel étant limitée (module élastique compris entre 0.1 et 1 MPa), et

- la taille d'un œil pouvant varier d'un patient à un autre,

il peut être avantageux de disposer de plusieurs modèles de blocs de gel pour permette une adaptation optimale du bloc de gel à toutes les tailles d'œil, sans devoir contraindre l'œil du patient.

C'est pourquoi les inventeurs ont développé trois modèles de blocs de gel :

- un premier modèle de bloc de gel dont la base inférieure circulaire concave présente un rayon de courbure de 7.1 millimètres,
- un deuxième modèle de bloc de gel dont la base inférieure circulaire concave présente un rayon de courbure de 7.6 millimètres,
- un troisième modèle de bloc de gel dont la base inférieure circulaire concave présente un rayon de courbure de 8.1 millimètres.

Bien entendu, le lecteur appréciera que plus (ou moins) de trois modèles de blocs de gel peuvent être prévus. Dans tous les cas, le choix du modèle de bloc de gel le plus adapté à un patient donné peut être réalisé par le praticien à partir d'examens standards de mesure d'œil préopératoires.

Des aspects préférés mais non limitatifs de l'interface de couplage sont les suivants :

- le bloc de gel peut être réalisé dans un matériau ayant un module élastique compris entre 0.1 et 1 MPa ;
- la hauteur du bloc de gel peut être sensiblement égale à la hauteur de la paroi latérale interne de la cloison à double-paroi ;

- la cloison à double-paroi et le bloc de gel peuvent être cylindriques, le diamètre du bloc de gel étant sensiblement égal au diamètre de la paroi latérale interne de la cloison à double-paroi, par exemple compris entre 11 et 14 millimètres ;
- la base inférieure du bloc de gel peuvent être concave, la distance entre les bases supérieure et inférieure :

  • étant comprise entre 1 millimètre et 2,5 millimètres au centre de la base inférieure,
  • étant sensiblement égale à 4,5 et 6 millimètres à la périphérie de la base inférieure ;

- la base inférieure du bloc de gel peut être concave, le rayon de courbure de la base inférieure étant compris entre 6,5 et 8,5 millimètres ;
- la hauteur de la paroi latérale externe peut être supérieure à la hauteur de la paroi latérale interne de sorte que le bord inférieur de la paroi latérale interne est plus proche de la fenêtre que le bord inférieur de la paroi latéral externe, le disque perforé reliant les bords inférieurs des parois latérales interne et externe ayant une forme sensiblement tronconique de sorte que les axes des lumières sont orientés vers un axe longitudinal de l'anneau ;
- la surface du disque perforé couverte par les lumières traversante peut être supérieure à la surface du disque perforé non couverte par les lumières traversantes ;
- l'anneau peut comprendre en outre une collerette tronconique ouverte au niveau de sa bordure de plus grand diamètre, ladite collerette étant reliée au bord supérieur de la paroi latérale externe par sa bordure de plus petit diamètre ;
- l'anneau peut comprendre en outre un rebord annulaire solidaire de la bordure de plus grand diamètre, ledit rebord s'étendant radialement vers l'extérieur et incluant une couche de caoutchouc souple sur sa face opposée à la collerette ;
- la cloison double-paroi, la collerette et le rebord annulaire peuvent être monoblocs.

L'invention concerne également un procédé d'installation d'une interface de couplage entre une source L.A.S.E.R. et un tissu à traiter, l'interface de couplage comprenant

- un anneau incluant :

  ◦ une cloison à double-paroi ayant des parois latérales interne et externe,
  ◦ un disque perforé s'étendant entre des bords inférieurs des parois latérales interne et externe,
  ◦ une fenêtre transparente à un faisceau L.A.S.E.R. montée sur les bords supérieurs des parois latérales interne et externe,

les parois latérales interne et externe, le disque perforé et la fenêtre définissant une chambre d'aspiration de l'anneau, et la fenêtre et la paroi latérale interne définissant également un logement de travail de l'anneau,

- un bloc de gel incluant un flanc latéral et des bases supérieure et inférieure,

le procédé comprenant les étapes suivantes :

- insertion du bloc de gel dans le logement de travail,
- positionnement de l'anneau contenant le bloc de gel au-dessus du tissu à traiter (centrage),
- mise en contact :

  ◦ du bord inférieur de la paroi latérale externe avec le tissu à traiter au niveau d'une région périphérique dudit tissu à traiter, et
  ◦ de la base inférieure du bloc de gel avec le tissu à traiter au niveau d'une région centrale dudit tissu à traiter,

- activation d'un dispositif d'aspiration pour créer une dépression dans la chambre d'aspiration, entre les parois latérales internes de l'anneau et le flanc latéral du bloc de gel,
- désactivation du dispositif d'aspiration dès que le seuil de dépression désiré est atteint,
- maintien de la dépression constante, pendant toute la durée de la procédure.

Une fois l'intervention terminée, l'interface de couplage est désolidarisée du tissu à traiter en rétablissant la pression atmosphérique dans la chambre d'aspiration.

**BREVE DESCRIPTION DES DESSINS**

D'autres caractéristiques et avantages de l'invention ressortiront clairement de la description qui en est réalisée ci-après, à titre indicatif et nullement limitatif, en référence aux figures annexées, dans lesquelles :

- la figure 1 est une représentation schématique d'une interface de couplage de l'art antérieur ;
- les figures 2 et 3 sont des représentations schématiques d'une interface de couplage selon l'invention,
- la figure 4 illustre un procédé d'installation de l'interface de couplage selon l'invention.

**EXPOSE DETAILLE DE L'INVENTION**

On va maintenant décrire l'interface de couplage selon l'invention en référence aux figures. Dans ces différentes figures, les éléments équivalents sont désignés par la même référence numérique.

L'interface de couplage est destinée à être disposée entre une source L.A.S.E.R. et une cible à traiter 2. La cible 2 est par exemple un tissu humain ou animal à traiter tel qu'un globe oculaire et plus précisément une cornée ou un cristallin.

Dans la suite de la description, l'invention sera décrite, à titre d'exemple, pour le traitement d'une cornée d'un œil humain ou animal. Néanmoins, il est bien évident pour l'homme du métier que l'interface de couplage selon l'invention peut être utilisée dans d'autres applications.

*1. Description générale de l'interface de couplage*

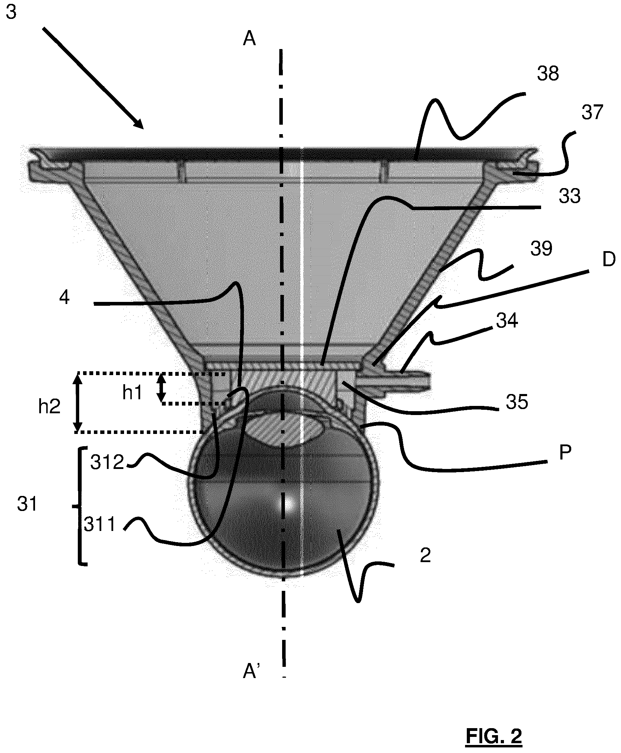

En référence à la figure 2, l'interface de couplage comprend :

- un anneau 3 s'étendant selon un axe longitudinal A-A' et ayant des extrémités proximale P et distale D, et
- un bloc de gel 4 présentant une forme générale cylindrique.

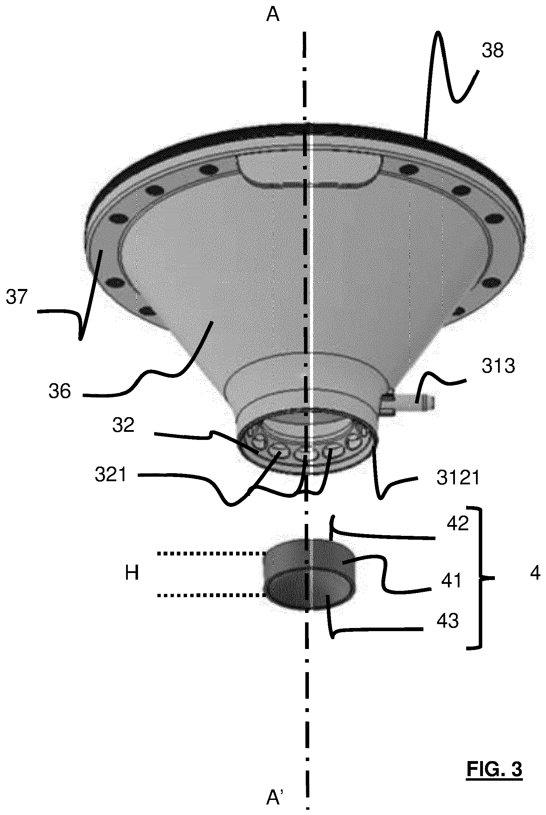

L'anneau 3 comporte : une cloison à double-paroi 31 ayant des parois interne et externe 311, 312, un disque perforé 32 (figure 3) à l'une des extrémités de la cloison 31, une fenêtre 33 transparente au rayonnement L.A.S.E.R. à l'autre extrémité de la cloison 31, et un canal d'aspiration 34 débouchant entre les parois 311, 312 de la cloison 31. La cloison à double-paroi 31 est agencée de sorte que seul le bord inférieur 3121 de la paroi externe 312 s'étende en saillie vers l'extérieur de l'anneau 3. Ce bord inférieur 3121 forme l'extrémité proximale P de l'anneau 3. Plus précisément, le bord inférieur 3121 de la paroi latérale externe 312 constitue l'unique arrête de l'anneau 3 destinée à venir en contact avec l'œil 2 du patient en périphérie du globe.

La fenêtre 33 est montée de manière étanche sur l'extrémité distale D de l'anneau 3 de sorte à fermer l'extrémité distale D. L'anneau 3 n'est donc ouvert qu'à son extrémité proximale P destinée à venir en contact avec l'œil 2 du patient.

La cloison 31, le disque 32 et la fenêtre 33 délimitent deux espaces à l'intérieur de l'anneau :

- une chambre d'aspiration 35 circulaire dans laquelle une dépression peut être créée en raccordant une unité d'aspiration au canal d'aspiration 34, et
- un logement de travail 36 adapté pour recevoir le bloc de gel 4, et à travers lequel les faisceaux L.A.S.E.R. émis par une source L.A.S.E.R. sont appliqués au tissu 2 à traiter.

Pour fixer l'interface de couplage sur l'œil 2 du patient, le bloc de gel 4 est monté dans le logement de travail 36, l'anneau 3 et le bloc de gel 4 sont positionnés sur l'œil 2 du patient, et une dépression est réalisée dans la chambre d'aspiration 35.

Avant l'application de la dépression, l'œil 2 du patient est en contact avec l'interface patient :

- au niveau du bloc de gel 4, et
- au niveau du bord inférieur 3121 de la paroi latérale externe 312 (qui constitue l'unique arrête en contact avec l'œil 2 du patient).

Ainsi et contrairement aux interfaces de couplage existantes, la fixation de l'interface de couplage sur l'œil 2 par la génération d'une dépression, ne nécessite pas d'avoir un appui parfait entre deux bordures circulaires définissant une gorge annulaire.

En raison de cette nouvelle conception (i.e. unique bordure circulaire 3121 et présence d'un bloc de gel 4 déformable), la génération de la dépression est simplifiée et plus agréable pour le patient puisque c'est l'interface de couplage qui se conforme à la configuration de l'œil (via la déformation du bloc de gel) et non l'oeil qui se conforme à la configuration de l'interface patient (comme c'est le cas avec le dispositif illustré à la figure 1).

On va maintenant décrire plus en détails l'interface de couplage selon l'invention.

*2. Description détaillée de l'interface de couplage*

*2.1. Anneau*

En référence aux figures 2 et 3, on a illustré l'anneau 3 comportant la cloison à double-paroi 31, le disque perforé 32, la fenêtre transparente 33 et le canal d'aspiration 34.

*2.1.1. Cloison à double-paroi*

La cloison à double-paroi 31 est de forme générale cylindrique, et est ouverte à ses deux extrémités.

Plus précisément, la cloison à double-paroi 31 est composée d'une paroi latérale interne 311 et d'une paroi latérale externe 312. Chaque paroi latérale 311, 312 inclut des bords supérieur et inférieur respectifs. Comme décrit précédemment, le bord inférieur 3121 de la paroi externe 312 est destiné à venir en contact avec l'œil 2 du patient. C'est pourquoi le bord inférieur 3121 de la paroi latérale externe 312 peut avantageusement être évasé afin de se conformer au mieux à la forme d'un œil 2. Le bord inférieur 3121 évasé de la paroi externe 312 peut (par exemple) présenter une forme générale tronconique incurvée vers l'extérieur apte à être appliquée sur la surface externe de l'œil 2 de manière non traumatisante. En référence à la figure 2, ce bord inférieur 3121 évasé présente un profil concave de rayon de courbure sensiblement égal au rayon de courbure de l'œil 2 afin de permettre un appui tangentiel.

Comme illustré à la figure 3, le bord inférieur de la paroi latérale interne 311 est en retrait vers l'intérieur de l'anneau 3. En particulier, le bord inférieur 3121 de la paroi latérale externe 312 s'étend en saillie vers l'extérieur, tandis que le bord inférieur de la paroi latérale interne 311 est décalé selon l'axe longitudinal A-A' vers l'intérieur de l'anneau 3.

Notamment, la hauteur h1 de la paroi latérale interne 311 est inférieure à la hauteur h2 de la paroi latérale externe 312, de sorte que le bord inférieur de la paroi latérale interne 311 est plus proche de la fenêtre 33 que le bord inférieur 3121 de la paroi latéral externe 312.

Ainsi, l'interface de couplage comprend une unique arrête circulaire destinée à venir en contact avec l'œil 2 du patient. Le fait que l'interface de couplage comprenne une unique arrête circulaire de contact (plutôt que deux bordures circulaires comme proposé dans les interfaces de couplage traditionnelles telles qu'illustrées à la figure 1) permet de faciliter l'obtention d'un contact parfait sur toute la circonférence de la ligne de contact entre l'oeil 2 et l'anneau 3.

### 2.1.2. *Disque perforé*

Le disque perforé 32 permet de relier les parois latérales interne et externe 311, 312 de la cloison à double-paroi 31. Il comprend des lumières traversantes 321 ou logettes destinées à coopérer avec la sclère de l'oeil 2 du patient lorsque l'interface de couplage est positionnée sur l'oeil 2 du patient et qu'une dépression est générée dans la chambre d'aspiration 35, de manière à ce que la membrane conjonctivale soit légèrement aspirée à l'intérieur de ces logettes formant ainsi un ensemble solidaire réduisant le risque de déventousage.

Dans le mode de réalisation illustré à la figure 3, le disque perforé 32 a une forme sensiblement tronconique de sorte que les axes des lumières du disque 32 sont orientés vers l'intérieur de l'anneau 3. Bien entendu, le disque perforé 32 peut présenter d'autres formes. Par exemple, le disque perforé 32 peut présenter la forme d'un tore tronqué. Dans ce cas, le rayon de courbure du disque perforé 32 est choisi dans une gamme comprise entre 5 et 10 millimètres, afin d'optimiser la conformation du disque 32 à l'œil 2 du patient.

Le nombre et la forme des lumières traversantes 321 peuvent varier en fonction de l'application visée. De préférence la surface du disque 32 couverte par les lumières 321 est supérieure à la surface du disque 32 non couverte par les lumières 321. Ceci permet de maximiser la surface de l'oeil sur laquelle la force d'aspiration s'applique, et donc de minimiser le niveau de vide (i.e. l'intensité de la dépression) nécessaire pour assurer le maintien en position de l'interface de couplage sur l'oeil 2 du patient.

### 2.1.3. *Fenêtre*

En référence à la figure 2, la fenêtre 33 transparente au faisceau généré par la source L.A.S.E.R. est montée de manière étanche sur les bords supérieurs des parois latérales interne et externe 311, 312 de la cloison à double-paroi 31.

La fenêtre 33 présente la forme d'un disque.

Bien entendu, la fenêtre 33 peut présenter d'autres formes (carrée, rectangulaire, elliptique) en fonction de l'application visée.

La fenêtre 33 peut être conçue en différents matériaux tels que du verre ou du plastique (Polycarbonate, Poly(méthacrylate de méthyle), etc.).

Dans le mode de réalisation illustré aux figures 2 et 3, la fenêtre 33 et la cloison à double-paroi 31 sont constituées en deux pièces distinctes. Néanmoins, dans certains modes de réalisation, la fenêtre 33 et la cloison à double-paroi 31 peuvent être constituées en une seule pièce (monobloc). Ceci permet de limiter les risques de fuite à la jonction entre la cloison à double-paroi 31 et la fenêtre 33.

Lorsque la fenêtre 33 et la cloison à double-paroi 31 sont deux pièces distinctes de l'anneau 3, la fenêtre 33 peut être fixée à la cloison à double-paroi 31 par collage, soudage ou toute autre technique permettant la fixation de manière étanche de la fenêtre 33 sur la cloison à double-paroi 31.

La fenêtre 33 peut être traitée antireflet ou présenter tout autre type de traitement optique afin d'améliorer la transmission du faisceau L.A.S.E.R. en fonction de sa longueur d'onde.

### 2.1.4. *Canal d'aspiration*

L'anneau 3 comprend également un canal tubulaire 34 traversant ménagé dans la paroi latérale externe 312 de la cloison à double-paroi 31. Ce canal - dit « *canal d'aspiration* » - débouche entre les parois interne et externe 311, 312 de la cloison à double-paroi 31, et s'étend radialement vers l'extérieur perpendiculairement à l'axe A-A'.

Le canal d'aspiration 34 permet le raccordement d'un dispositif distant à l'interface de couplage par l'intermédiaire d'une tubulure. En particulier, le canal d'aspiration 34 permet le raccordement de l'interface de couplage à un dispositif d'aspiration pour la génération d'une dépression dans la chambre d'aspiration 35 définie entre la fenêtre 33, les parois latérales interne et externe 311, 312 , et le disque perforé 32.

La présence d'un unique canal 34 permet de limiter l'encombrement de l'interface de couplage et de réduire le nombre de tubulures et de connecteurs.

### 2.1.5. *Autres éléments optionnels de l'anneau*

Outre les éléments décrits ci-dessus, l'anneau 3 peut également comprendre une collerette tronconique 39 reliée au bord supérieur de la paroi latérale externe 312 par sa bordure de plus petit diamètre.

Une telle collerette 39 est ouverte au niveau de sa bordure de plus grand diamètre, et est solidaire d'un rebord annulaire 37 s'étendant radialement vers l'extérieur de la collerette 39.

Avantageusement, ce rebord annulaire 37 comprend une couche de caoutchouc souple 38 sur sa face opposée à la collerette 39. Ceci permet de faciliter la fixation de la source L.A.S.E.R. à l'interface de couplage par ventousage. En effet, même si plusieurs solutions (mécanique, magnétique, etc.) peuvent être envisagées pour solidariser la source L.A.S.E.R. à l'interface de couplage, il est préférable d'utiliser des moyens de fixation par dépression afin de limiter les risques « *d'à coup* » lors de cette solidarisation.

### 2.2.Bloc de gel

L'interface de couplage comprend également un bloc de gel 4 destiné à coopérer avec l'anneau 3.

En référence à la figure 3, le bloc de gel 4 présente une forme générale cylindrique. Il comprend :

- un flanc latéral 41 de forme complémentaire à la forme de la paroi latérale interne,
- une base supérieure 42 circulaire sensiblement plane,
- une base inférieure 43 circulaire concave.

Le bloc de gel 4 est destiné à être positionné dans le logement de travail 36 de l'anneau 3. Plus précisément, le flanc latéral 41 du bloc de gel 4 est destiné à venir en contact avec la face intérieure de la paroi latérale interne 311, et la base supérieure 42 est destinée à venir en contact avec la fenêtre 33, tandis que la base inférieure 43 est destinée à venir en contact avec l'oeil 2 du patient.

C'est pourquoi la base inférieure 43 présente une concavité définie par un rayon de courbure choisi dans une gamme comprise entre 5 et 10 millimètres, préférentiellement entre 6,5 et 8,5 millimètres. Ceci permet d'optimiser la conformation de la base inférieure à l'œil 2 du patient.

Avantageusement, le bloc de gel 4 peut être un hydrogel ou tout autre matériau souple transparent biocompatible et stérilisable, aux propriétés optiques compatibles avec la propagation d'un laser femtoseconde. Cet hydrogel peut par exemple comprendre un mélange de fluorosilicone et de monomères hydrophiles. Diverses variantes d'hydrogel peuvent être utilisées, comme par exemple des hydrogels présentant une teneur en eau supérieure à 70%, ou comprise entre 50% et 70%, ou comprise entre 30 et 50%. Ceci permet de disposer de bloc de gel ayant des propriétés lubrifiantes et optiques différentes, ainsi que des compressibilités différentes.

Dans tous les cas, les dimensions et la forme du bloc de gel 4 sont déterminées de sorte à ce que le bloc de gel 4 coopère intimement avec le logement de travail 36.

Notamment, la hauteur H du bloc de gel 4 est de préférence sensiblement égale - voire légèrement supérieure - à la hauteur h1 de la paroi latérale interne 311. Ceci permet de limiter les risques de déplacement du bloc de gel vers la chambre d'aspiration 35 lors de la génération de la dépression, celui-ci étant comprimé entre le tissu et la fenêtre lors de la génération de la dépression. On réduit ainsi les risques de décentrage du bloc de gel. Ceci permet également d'éviter la présence de bulle :

- entre le bloc de gel 4 et la fenêtre 33 et/ou
- entre le bloc de gel 4 et l'oeil 2 du patient,

une fois l'interface de couplage plaquée sur l'œil 2 à traiter.

Par ailleurs, le diamètre du bloc de gel 4 est de préférence sensiblement égal - voire légèrement inférieur - au diamètre de la paroi latérale interne 311 (entre 11 et 14 millimètres). Ceci permet de faciliter l'insertion du bloc de gel dans le logement de travail en assurant sa mobilité en translation selon l'axe A-A'.

Enfin, pour que le bloc de gel 4 assure le maintien en position de l'œil 2 du patient, la distance entre les bases supérieure et inférieure du bloc de gel 4 peut :

- être comprise entre 1 millimètre et 2,5 millimètres au centre de la base inférieure circulaire,
- être sensiblement égale à 4,5 et 6 millimètres à la périphérie de la base inférieure.

### 3. Principe de fonctionnement

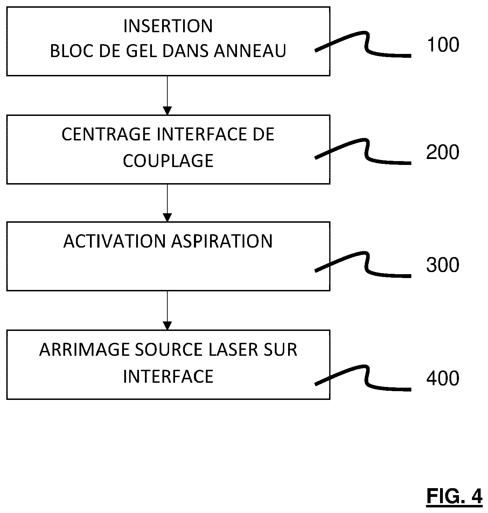

On va maintenant décrire plus en détail le principe de fonctionnement de l'interface de couplage selon l'invention en référence à l'interface de couplage représentée aux figures 2 et 3, et au procédé illustré à la figure 4.

On suppose que le canal d'aspiration 34 a été préalablement raccordé au dispositif d'aspiration (non représenté) par l'intermédiaire d'une tubulure.

Dans une première étape 100, le praticien insère (en conditions stériles) le bloc de gel 4 dans le logement de travail 36 et le pousse au fond du logement jusqu'à ce que la partie plane distale du bloc de gel vienne se coller à la fenêtre 33.

Dans une deuxième étape 200, le praticien positionne l'anneau 3 et le bloc de gel 4 au-dessus de l'œil 2, de sorte que le centre de l'œil et l'ensemble composé de l'anneau et du bloc de gel 4 soient alignés. Lorsque l'interface de couplage (anneau + bloc de gel) est centré sur l'œil du patient, le praticien met en contact le bord inférieur 3121 de la paroi latérale externe 312 avec la périphérie de l'œil 2 du patient et plus précisément sur une surface du globe recouverte par la membrane conjonctivale.

Lorsque l'interface de couplage est correctement centrée sur l'œil 2 du patient, le praticien active le dispositif d'aspiration (étape 300). L'activation du dispositif d'aspiration permet de créer une dépression dans la chambre d'aspiration 35, ce qui exerce le ventousage. Du fait de la génération de la dépression, la membrane conjonctivale recouvrant la sclère de l'œil 2 est aspirée au niveau des lumières traversantes 321 du disque perforé 32. Ceci permet de limiter les mouvement relatifs de l'œil 2 par rapport à l'interface de couplage. L'orientation des lumières traversantes 321 (dont les axes de symétrie sont orientés vers l'axe A-A') permet l'application d'un effort tangentiel sur l'œil 2 du patient tendant à immobiliser celui-ci.

L'œil 2 se plaque contre le bord inférieur 3121 de la paroi latérale externe 312 d'une part, et contre le bloc de gel 4 d'autre part. Du fait de sa malléabilité, le bloc de gel épouse parfaitement la forme de l'oeil 2 du patient.

Une fois l'interface de couplage ventousée sur l'œil 2, le praticien commande l'arrimage de la source L.A.S.E.R. sur l'interface de couplage, tout en maintenant le dispositif d'aspiration activé (étape 400). La fixation de la source L.A.S.E.R. (qui peut par exemple être montée sur un bras articulé tel que décrit dans le document WO 2019/145487) à l'interface de couplage peut avantageusement être réalisée par ventousage pour limiter tout déplacement inopiné de l'interface de couplage.

### 4. Conclusions

Les interventions chirurgicales effectuées en ophtalmologie et ayant recours à une source L.A.S.E.R. (notamment femtoseconde), utilisent habituellement un système de maintien du globe oculaire, qui doit être actif pendant toute la durée d'exposition du patient au faisceau L.A.S.E.R..

En effet, le risque serait qu'en cas de mouvement inopiné et non maîtrisé du globe oculaire, le faisceau atteigne des zones non supposées être atteintes et génère des lésions plus ou moins graves des structures intraoculaires.

L'interface de coupage décrite précédemment permet un maintien optimal du globe oculaire, et assure au praticien une connaissance précise de la position dans l'espace du globe afin de diriger précisément le faisceau L.A.S.E.R. sur sa cible.

Le lecteur aura compris que de nombreuses modifications peuvent être apportées à l'invention décrite précédemment sans sortir matériellement des nouveaux enseignements et des avantages décrits ici.

### Revendications

1. Interface de couplage entre une source L.A.S.E.R. et un tissu à traiter (2), l'interface de couplage comprenant :

   - un anneau (3) ayant une extrémité proximale (P) destinée à venir en contact avec le tissu (2) à traiter et une extrémité distale (D) opposée, l'anneau (3) incluant :

      • une cloison à double-paroi (31) comportant :

         ■ une paroi latérale interne (311) ayant un bord supérieur et un bord inférieur, et

         ■ une paroi latérale externe (312) ayant un bord supérieur et un bord inférieur, le bord inférieur de la paroi latérale interne (311) étant plus proche de l'extrémité distale (D) que le bord inférieur (3121) de la paroi latérale externe (312), et les bords supérieurs des parois latérales interne et externe (311, 312) étant plus proches de l'extrémité distale (D) que les bords inférieurs des parois latérales interne et externe (311, 312),

      • un disque perforé (32) s'étendant entre les bords inférieurs des parois latérales interne et externe (311, 312), le disque perforé (32) incluant des lumières traversantes (321),
      • une fenêtre (33) transparente à un faisceau L.A.S.E.R. généré par la source L.A.S.E.R., ladite fenêtre (33) étant montée de manière étanche sur les bords supérieurs des parois latérales interne et externe (311, 312) de sorte à fermer l'extrémité distale (D) de l'anneau (3), la fenêtre (33) définissant :

         ■ une chambre d'aspiration (35) périphérique avec le disque perforé (32), la paroi latérale interne (311) et la paroi latérale externe (312), et
         ■ un logement de travail (36) central avec la paroi latérale interne (311), et

      • un canal d'aspiration (34) s'étendant entre les faces intérieure et extérieure de la paroi latérale externe (312) de sorte à déboucher dans la chambre d'aspiration (35), le canal d'aspiration (34) étant destiné à être connecté à un module d'aspiration pour permettre la génération d'une dépression dans un espace intérieur défini par la chambre d'aspiration (35),

   - un bloc de gel (4) destiné à être monté de manière amovible dans le logement de travail (36), ledit bloc de gel (4) étant apte à coulisser le long de la paroi latérale interne de la cloison à double-paroi (31) et ayant :

      • un flanc latéral (41) de forme complémentaire à la forme de la paroi latérale interne (311), le flanc latéral (41) étant destiné à venir en contact avec la face intérieure de la paroi latérale interne (311),
      • une base supérieure (42) circulaire sensiblement plane, la base supérieure (42) étant destinée à venir en contact avec la fenêtre (33),
• une base inférieure (43), la base inférieure (43) étant destinée à venir en contact avec le tissu (2) à traiter.

2. Interface de couplage selon la revendication 1, *dans laquelle* la hauteur (H) du bloc de gel (4) est sensiblement égale à la hauteur (h1) de la paroi latérale interne (311) de la cloison à double-paroi (31).

3. Interface de couplage selon l'une quelconque des revendications précédentes, *dans laquelle* la cloison à double-paroi (31) et le bloc de gel (4) sont cylindriques, le diamètre du bloc de gel (4) étant sensiblement égal au diamètre de la paroi latérale interne (311) de la cloison à double-paroi (31), par exemple compris entre 11 et 14 millimètres.

4. Interface de couplage selon l'une quelconque des revendications précédentes, *dans laquelle* la base inférieure (43) du bloc de gel (4) est concave, la distance entre les bases supérieure (42) et inférieure (43) :

   - étant comprise entre 1 millimètre et 2,5 millimètres au centre de la base inférieure (43),
   - étant sensiblement égale à 4,5 et 6 millimètres à la périphérie de la base inférieure (43).

5. Interface de couplage selon l'une quelconque des revendications précédentes, *dans laquelle* la base inférieure (43) du bloc de gel (4) est concave, le rayon de courbure de la base inférieure (43) étant compris entre 6,5 et 8,5 millimètres.

6. Interface de couplage selon l'une quelconque des revendications précédentes, *dans laquelle* la hauteur (h2) de la paroi latérale externe (312) est supérieure à la hauteur (h1) de la paroi latérale interne (311) de sorte que le bord inférieur de la paroi latérale interne (311) est plus proche de la fenêtre (33) que le bord inférieur (3121) de la paroi latéral externe (312), le disque perforé (32) reliant les bords inférieurs des parois latérales interne et externe (311, 312) ayant une forme sensiblement tronconique de sorte que les axes des lumières (321) sont orientés vers un axe longitudinal (A-A') de l'anneau (3).

7. Interface de couplage selon l'une quelconque des revendications précédentes, *dans laquelle* la surface du disque perforé (32) couverte par les lumières traversante (321) est supérieure à la surface du disque perforé (32) non couverte par les lumières traversantes (321).

8. Interface de couplage selon l'une quelconque des revendications précédentes, *dans laquelle* l'anneau (3) comprend en outre une collerette tronconique (39) ouverte au niveau de sa bordure de plus grand diamètre, ladite collerette (39) étant reliée au bord supérieur de la paroi latérale externe (312) par sa bordure de plus petit diamètre.

9. Interface de couplage selon la revendication précédente, *dans laquelle* l'anneau (3) comprend en outre un rebord annulaire (37) solidaire de la bordure de plus grand diamètre, ledit rebord (37) s'étendant radialement vers l'extérieur et incluant une couche de caoutchouc souple (38) sur sa face opposée à la collerette (39).

10. Interface de couplage selon la revendication précédente, *dans laquelle* la cloison double-paroi (31), la collerette (39) et le rebord annulaire (37) sont monoblocs.

**Patentansprüche**

1. Koppelschnittstelle zwischen einer Laserquelle und einem zu behandelnden Gewebe (2), wobei die Koppelschnittstelle umfasst:

   - einen Ring (3), der ein proximales Ende (P), das dazu bestimmt ist, mit dem zu behandelnden Gewebe (2) in Kontakt zu kommen, und ein entgegengesetztes distales Ende (D), aufweist, wobei der Ring (3) umfasst:

     • eine doppelwandige Trennwand (31) umfassend:

       • eine innenliegende Seitenwand (311), die eine obere Kante und eine untere Kante aufweist, und
       • eine außenliegende Seitenwand (312), die eine obere Kante und eine untere Kante aufweist, wobei die untere Kante der innenliegenden Seitenwand (311) näher an dem distalen Ende (D) als die untere Kante (3121) der außenliegenden Seitenwand (312) liegt, und die oberen Kanten der innenliegenden und außenliegenden Seitenwände (311, 312) näher an dem distalen Ende (D) als die unteren Kanten der innenliegenden und außenliegenden Seitenwände (311, 312) liegen,

     • eine Lochscheibe (32), die sich zwischen den unteren Kanten der innenliegenden und außenliegenden Seitenwände (311, 312) erstreckt, wobei die Lochscheibe (32) durchgehende Öffnungen (321) umfasst,

• ein Fenster (33), das für einen Laserstrahl durchsichtig ist, der von der Laserquelle erzeugt wird, wobei das Fenster (33) an den oberen Kanten der innenliegenden und außenliegenden Seitenwände (311, 312) dicht montiert ist, um das distale Ende (D) des Rings (3) zu schließen, wobei das Fenster (33):

• eine Ansaugkammer (35), die zu der Lochscheibe (32), der innenliegenden Seitenwand (311) und der außenliegenden Seitenwand (312) peripherisch ist, und
• eine Arbeitsaufnahme (36), die zu der innenliegenden Seitenwand (311) in der Mitte liegt, definiert, und

• einen Ansaugkanal (34), der sich zwischen den inneren und äußeren Seiten der außenliegenden Seitenwand (312) erstreckt, so dass er in die Ansaugkammer (35) einmündet, wobei der Ansaugkanal (34) dazu bestimmt ist, mit einem Ansaugmodul verbunden zu sein, um das Erzeugen eines Unterdrucks in einem inneren Raum zu ermöglichen, der durch die Ansaugkammer (35) definiert ist,

- einen Gelblock (4), der dazu bestimmt ist, in der Arbeitsaufnahme (36) abnehmbar montiert zu sein, wobei der Gelblock (4) dazu geeignet ist, entlang der innenliegenden Seitenwand der doppelwandigen Trennwand (31) zu gleiten, und:

• eine seitliche Flanke (41), deren Form die Form der innenliegenden Seitenwand (311) ergänzt, wobei die seitliche Flanke (41) dazu bestimmt ist, mit der inneren Seite der innenliegenden Seitenwand (311) in Kontakt zu kommen,
• eine kreisförmige, im Wesentlichen ebene obere Basis (42), wobei die obere Basis (42) dazu bestimmt ist, mit dem Fenster (33) in Kontakt zu kommen,
• eine untere Basis (43), wobei die untere Basis (43) dazu bestimmt ist, mit dem zu behandelnden Gewebe (2) in Kontakt zu kommen, aufweist.

2. Koppelschnittstelle nach Anspruch 1, wobei die Höhe (H) des Gelblocks (4) im Wesentlichen gleich der Höhe (h1) der innenliegenden Seitenwand (311) der doppelwandigen Trennwand (31) ist.

3. Koppelschnittstelle nach einem der vorhergehenden Ansprüche, wobei die doppelwandige Trennwand (31) und der Gelblock (4) zylindrisch sind, wobei der Durchmesser des Gelblocks (4) im Wesentlichen gleich dem Durchmesser der innenliegenden Seitenwand (311) der doppelwandigen Trennwand (31) ist, zum Beispiel zwischen 11 und 14 Millimetern liegt.

4. Koppelschnittstelle nach einem der vorhergehenden Ansprüche, wobei die untere Basis (43) des Gelblocks (4) konkav ist, wobei der Abstand zwischen der oberen (42) und der unteren (43) Basis:

- zwischen 1 Millimeter und 2,5 Millimetern in der Mitte der unteren Basis (43) liegt,
- im Wesentlichen gleich 4,5 und 6 Millimeter an der Peripherie der unteren Basis (43) ist.

5. Koppelschnittstelle nach einem der vorhergehenden Ansprüche, wobei die untere Basis (43) des Gelblocks (4) konkav ist, wobei der Krümmungsradius der unteren Basis (43) zwischen 6,5 und 8,5 Millimetern liegt.

6. Koppelschnittstelle nach einem der vorhergehenden Ansprüche, wobei die Höhe (h2) der außenliegenden Seitenwand (312) größer als die Höhe (h1) der innenliegenden Seitenwand (311) ist, so dass die untere Kante der innenliegenden Seitenwand (311) näher an dem Fenster (33) als die untere Kante (3121) der außenliegenden Seitenwand (312) liegt, wobei die Lochscheibe (32), welche die unteren Kanten der innenliegenden und außenliegenden Seitenwände (311, 312) verbindet, im Wesentlichen kegelstumpfförmig ist, so dass die Achsen der Öffnungen (321) zu einer Längsache (A-A') des Rings (3) hin orientiert sind.

7. Koppelschnittstelle nach einem der vorhergehenden Ansprüche, wobei die Oberfläche der Lochscheibe (32), die mit den durchgehenden Öffnungen (321) bedeckt ist, größer ist als die Oberfläche der Lochscheibe (32), die nicht mit den durchgehenden Öffnungen (321) bedeckt ist.

8. Koppelschnittstelle nach einem der vorhergehenden Ansprüche, wobei der Ring (3) ferner einen kegelstumpfförmigen Kragen (39) umfasst, der an seiner Einfassung mit dem größten Durchmesser offen ist, wobei der Kragen (39) an der oberen Kante der außenliegenden Seitenwand (312) durch seine Einfassung mit dem kleinsten Durchmesser verbunden ist.

9. Koppelschnittstelle nach dem vorhergehenden Anspruch, wobei der Ring (3) ferner einen ringförmigen Rand (37) umfasst, der mit der Einfassung mit dem größten Durchmesser fest verbunden ist, wobei sich der Rand (37) radial nach außen erstreckt und eine nachgiebige Gummischicht (38) auf seiner Seite, die dem Kragen (39) gegenüberliegt umfasst.

10. Koppelschnittstelle nach dem vorhergehenden Anspruch, wobei die doppelwandige Trennwand (31), der Kragen (39) und der ringförmige Rand (37) einstückig sind.

**Claims**

1. A coupling interface between a laser source and a tissue to be treated (2), the coupling interface comprising:

    - a ring (3) having a proximal end (P) for contacting the tissue (2) to be treated and an opposite distal end (D), the ring (3) including:

        • a double-walled partition (31) having:

            ▪ an inner side wall (311) having an upper edge and a lower edge, and
            ▪ an outer side wall (312) having an upper edge and a lower edge, the lower edge of the inner side wall (311) being closer to the distal end (D) than the lower edge (3121) of the outer side wall (312), and the upper edges of the inner and outer side walls (311, 312) being closer to the distal end (D) than the lower edges of the inner and outer side walls (311, 312),

        • a perforated disc (32) extending between the lower edges of the inner and outer side walls (311, 312), the perforated disc (32) including through lumens (321),
        • a window (33) transparent to a laser beam generated by the laser source, said window (33) being sealingly mounted to the upper edges of the inner and outer side walls (311, 312) so as to close the distal end (D) of the ring (3), the window (33) defining:

            ▪ an aspiration chamber (35) peripheral to the perforated disc (32), the inner side wall (311) and the outer side wall (312), and
            ▪ a working housing (36) central with the inner side wall (311), and

        • an aspiration channel (34) extending between the inside and outside faces of the outer side wall (312), so as to open into the aspiration chamber (35), the aspiration channel (34) being intended to be connected to an aspiration module for allowing the generation of a vacuum in an interior space defined by the aspiration chamber (35),

    - a gel block (4) intended to be removably mounted in the working housing (36), said gel block (4) being able to slide along the inner side wall of the double-walled partition (31) and having:

        • a side flank (41) with a shape complementary to the shape of the inner side wall (311), the side flank (41) being intended to come into contact with the inside face of the inner side wall (311),
        • a substantially planar circular upper base (42), the upper base (42) being intended to come into contact with the window (33),
        • a lower base (43), the lower base (43) being intended to come into contact with the tissue (2) to be treated.

2. The coupling interface according to claim 1, *wherein* the height (H) of the gel block (4) is substantially equal to the height (h1) of the inner side wall (311) of the double-walled partition (31).

3. The coupling interface according to any one of the preceding claims, *wherein* the double-walled partition (31) and the gel block (4) are cylindrical, the diameter of the gel block (4) being substantially equal to the diameter of the inner side wall (311) of the double-walled partition (31), for example between 11 and 14 millimetres.

4. The coupling interface according to any one of the preceding claims, *wherein* the lower base (43) of the gel block (4) is concave, the distance between the upper base (42) and the lower base (43):

    - ranging between 1 millimetre and 2.5 millimetres at the centre of the lower base (43),
    - being substantially equal to 4.5 and 6 millimetres at the periphery of the lower base (43).

5. The coupling interface according to any one of the preceding claims, *wherein* the lower base (43) of the gel block (4) is concave, the radius of curvature of the lower base (43) ranging between 6.5 and 8.5 millimetres.

6. The coupling interface according to any one of the preceding claims, *wherein* the height (h2) of the outer side wall (312) is greater than the height (h1) of the inner side wall (311) so that the lower edge of the inner side wall (311) is closer to the window (33) than the lower edge (3121) of the outer side wall (312), the perforated disc (32) connecting the lower edges of the inner and outer side walls (311, 312) having a substantially frustoconical shape so that the axes of the lumens (321) are oriented towards a longitudinal axis (A-A') of the ring (3).

7. The coupling interface according to any one of the preceding claims, *wherein* the surface area of the perforated disc (32) covered by the through lumens (321) is greater than the surface area of the perforated disc (32) not covered by the through lumens (321).

8. The coupling interface according to any one of the preceding claims, *wherein* the ring (3) further comprises a frustoconical flange (39) open at its largest diameter rim, said flange (39) being connected to the upper edge of the outer side wall (312) by its smallest diameter rim.

9. The coupling interface according to the preceding claim, *wherein* the ring (3) further comprises an annular brim (37) integral with the largest diameter rim, said brim (37) extending radially outwards and including a flexible rubber layer (38) on its face opposite the flange (39).

10. The coupling interface according to the preceding claim, *wherein* the double-walled partition (31), the flange (39) and the annular brim (37) are in one piece.

**FIG. 1**

**FIG. 2**

A

38

37

36

313

32

321

3121

H

42

41

4

43

**FIG. 3**

A'

```
┌─────────────────────────────┐
│          INSERTION          │
│   BLOC DE GEL DANS ANNEAU   │        100
└─────────────────────────────┘
              │
              ▼
┌─────────────────────────────┐
│   CENTRAGE INTERFACE DE      │
│         COUPLAGE             │        200
└─────────────────────────────┘
              │
              ▼
┌─────────────────────────────┐
│    ACTIVATION ASPIRATION     │        300
└─────────────────────────────┘
              │
              ▼
┌─────────────────────────────┐
│  ARRIMAGE SOURCE LASER SUR   │
│         INTERFACE            │        400
└─────────────────────────────┘
```

**FIG. 4**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2018154038 A
- DE 102005040338
- WO 2019104256 A
- WO 2019145487 A